(19) Europäisches Patentamt
European Patent Office
Office européen des brevets

(11) Publication number: **0 164 026 A1**

(12) # EUROPEAN PATENT APPLICATION

(21) Application number: **85106310.7**

(22) Date of filing: **22.05.85**

(51) Int. Cl.[4]: **C 07 C 101/30**, C 07 C 99/12, C 07 C 143/20, C 07 B 57/00

(30) Priority: **29.05.84 GB 8413652**

(43) Date of publication of application: **11.12.85 Bulletin 85/50**

(84) Designated Contracting States: **BE CH DE FR GB IT LI NL SE**

(71) Applicant: **BEECHAM GROUP PLC, Beecham House Great West Road, Brentford Middlesex TW8 9BD (GB)**

(72) Inventor: **Richardson, Jill Margaret, 8 Sompting Road Lancing, West Sussex, BN15 9LB (GB)**

(74) Representative: **Lockwood, Barbara Ann et al, Beecham Pharmaceuticals Biosciences Research Centre Great Burgh Yew Tree Bottom Road, Epsom Surrey KT18 5XQ (GB)**

(54) **Process for the preparation of optically active glycine derivatives.**

(57) Resolution of dihydroxyphenylglycines, in particular 3,4-dihydroxyphenylglycine, by use of 3-bromocamphor-9-sulphonic acid (HSC).

EP 0 164 026 A1

ACTORUM AG

0 164 026



B1649

TITLE MODIFIED
see front page

CHEMICAL PROCESS

This invention relates to a chemical process and in particular to a process for the resolution of dihydroxyphenyl-glycines in order to isolate an optically active dihydroxyphenylglycine. The invention also relates to certain novel intermediates employed in the process.

Dihydroxyphenylglycines are α-amino acids which are useful as precursors for the synthesis of antibacterially active penicillin and cephalosporin derivatives. As such, the compounds are usually required in a single optically active form, particularly the D form, whereas most synthetic methods for the production of dihydroxyphenylglycines result in the racemic, or DL, form.

It has now been found that DL-dihydroxyphenylglycines may be separated into their optically active enantiomers by use of the diastereoisomeric salts with 3-bromocamphor-9-sulphonic acid.

Accordingly the present invention provides a diastereoisomeric 3-bromocamphor-9-sulphonate salt of a dihydroxyphenylglycine.

The compound 3-bromocamphor-9-sulphonic acid is also known as α-bromocamphor-π-sulphonic acid, which has on occasions been incorrectly designated as the 8-sulphonic acid.

The camphor sulphonate salts of this invention include those having the formula (I):

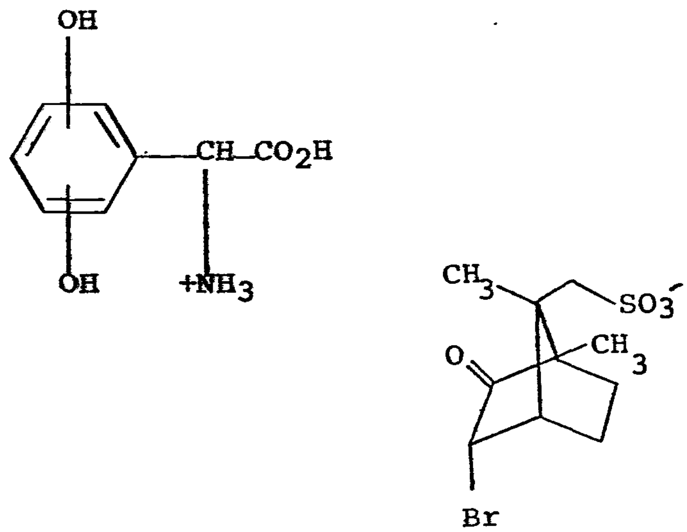

(I)

The camphorsulphonate salt of formula (I) may be prepared by
treating a racemic dihydroxyphenylglycine of formula (II):

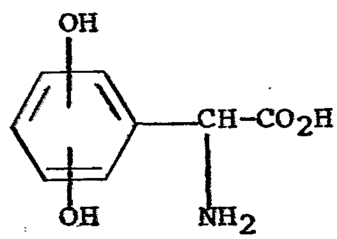

(II)

with optically active 3-bromocamphor-9-sulphonic acid of formula (III):

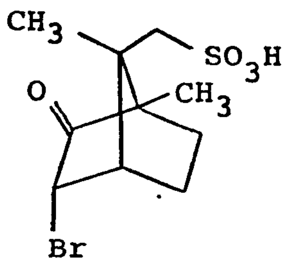

(III)

Suitable solvents for this reaction include polar solvents, such as water, $C_{1-6}$ alkanoic acids, such as acetic acid, or dilute mineral acids, such as dilute sulphuric acid.

It is preferred to employ the camphorsulphonic acid of formula (III) in the form of a salt thereof, such as the sodium or potassium salt, and liberate the acid by carrying out the reaction in the presence of a strong mineral acid.

The dihydroxy phenylglycines of formula (II) are known compounds and may be prepared by conventional methods such as reaction of a dihydroxybenzaldehyde with ammonia and hydrogen cyanide followed by hydrolysis; or by the process as described in British Patent No. 1,371,896.

Suitable dihydroxyphenylglycines of formula (II) include α-amino -2,4-dihydroxyphenylacetic acid, α-amino-2,5-dihydroxyphenylacetic acid, and α-amino-3,4-dihydroxyphenylacetic acid.

When the racemic dihydroxyphenylglycine of formula (II) is treated with the optically active camphorsulphonic acid, a mixture of two diastereoisomeric camphorsulphonate salts are produced, which have different solubilities.

One of these salts is then isolated by preferential crystallisation. It is preferred to carry out the crystallisation in the same solvent as the camphorsulphonate salt is prepared. The isolated salt is then cleaved with a strong acid or base to produce the optically active dihydroxyphenylglycine.

Accordingly in a further aspect, this invention provides a process for the preparation of an optically active dihydroxyphenylglycine, which process comprises treating a diastereomeric 3-bromocamphor-9-sulphonate salt of a dihydroxyphenylglycine with a base.

Furthermore, the present invention provides a process for the preparation of an optically active dihydroxyphenylglycine which process comprises reacting a racemic dihydroxyphenylglycine with an optically active 3-bromocamphor-9-sulphonic acid to yield two diastereoisomeric salts, isolating one salt by preferential crystallisation, and liberating the optically active dihydroxyphenylglycine therefrom.

The diastereoisomeric bromocamphorsulphonate salts are separated by preferential crystallisation. This may be induced by concentration of the solution, by adding a further solvent in which one diastereoisomer is less soluble, such as water; or by a seed crystal of the desired diastereoisomer.

After separation of the formed crystals the mother liquor can be treated to recover the other diastereoisomeric salt by further recrystallisation, prefereably after addition of a seed crystal of the diastereoisomeric salt remaining in solution in excess. The mother liquor may be conveniently treated

with a further quantity of 3-bromocamphor-9-sulphonic acid, whereupon the solution will contain an excess of the diasteroisomeric salt which did not first crystallise, and this salt will now crystallise out on evaporation, though preferably a seed crystal of such salt is added to induce crystallisation.

After isolation, the desired diastereoisomeric camphorsulphonate salt is treated with a strong base. Suitable bases are those which are capable of hydrolysing the dihydroxyphenylglycine salt to the dihydroxyphenylglycine, for example bases which are stronger than the dihydroxyphenylglycine, such as ammonia, sodium hydroxide and potassium hydroxide.

The liberated camphorsulphonic acid can be recovered and re-used.

In the process of this invention, it is preferred to employ the L(+) 3-bromocamphor-9-sulphonic acid, which causes preferential crystallisation of a salt with the D-enantiomer of the dihydroxyphenylglycine, the latter being the preferred enantiomer for use in the synthesis of penicillin and cephalosporin antibiotics.

Since it is the D-form of the dihydroxy phenylglycine which finds most use, particularly in the production of useful penicillins and cephalosporins, the L-form is not particularly desired. After separation this may be racemised to reform the DL-form, for example by treating the compound with hydrochloric acid at high temperature, for example 130$^{o}$-140$^{o}$C, whereafter the process of the invention can be repeated to produce quantities of the desired D-isomer.

The following Examples illustrate this invention.

Example 1

Precipitation of D-3,4-dihydroxyphenylglycine salt with 3-bromocamphor-9-sulphonic acid.

5g (0.0273 mole) of DL-3,4-dihydroxyphenylglycine was slurried in 200 ml of glacial acetic acid at 40°C, and 6.80g (0.0219 mole) of L(+)3-bromocamphor-9-sulphonic acid was added. Seed crystals of the salt of D-3,4 dihydroxyphenylglycine with L(+) 3 bromo-9-sulphonic acid were added. The mixture was stirred for 5 hours, the title salt filtered off, and dried overnight *in vacuo* at ambient temperature. The product was a white solid containing approx.38% 3,4-dihydroxyphenylglycine, indicating a 1:1 salt.

Yield: 46%

$[\alpha]_{MeOH}$ + 5°

Example 2

Cleavage of the salt and crystallisation of D-3,4-dihydroxyphenylglycine

4g (0.0081 mole) of D-3,4-dihydroxyphenylglycine 3-bromocamphor-9-sulphonate was dissolved in 10ml of methanol and 10% sodium hydroxide solution added to pH6. The slurry was stirred for a further 2 hours before filtering, washing with water and drying *in vacuo* overnight at ambient temperature. The product was a white crystalline solid.

Yield: 90%

$[\alpha]_{2NHCl}$ : $-151^{o}$

Example 3

D-3,4-dihydroxyphenylglycine salt with 3-bromocamphor-9-sulphonic acid

10.07 g (0.55 mole) DL-3,4-dihydroxyphenylglycine, 4.98 g (0.0286 mole) potassium sulphate, 5.39 g (0.055 mole) sulphuric acid, 0.035 mol potassium 3-bromo camphor-9-sulphonate (as a 30% aqueous solution) and sufficient water to make a total volume of 45 mls was stirred at $80^{o}C$ until all solids had dissolved.

The solution was then cooled to $65^{o}C$ slowly, seeded with D-3,4-dihydroxyphenylglycine/3-bromocamphor-9-sulphonate salt and allowed to cool to ambient with stirring for a total of 7 hours.

The precipitate was then filtered off to produce a white crystalline solid.

Yield 35% from DL-3,4-dihydroxyphenylglycine
$[\alpha]_{MeOH}+5^{o}$

The salt was cleaved as described in Example 2 to give D-3,4-dihydroxyphenylglycine.

Example 4

D-3,4-dihydroxyphenylglycine salt with 3-bromocamphor-9-sulphonic acid

10.07 g (0.055 mole) DL-3,4-dihydroxyphenylglycine was slurried in 45 mls water together with 13.66 g (0.044 mole) L(+)3-bromo camphor-9-sulphonic acid and 3 mls (0.055 mole) sulphuric acid was added. Seed crystals of the salt of D-3,4 dihydroxyphenyl glycine with 3 bromocamphor-9-sulphonic acid were added and the mixture stirred for 7 hours before filtering off the resulting salt.

Yield 35% from DL-3-4-dihydroxyphenylglycine
$[\alpha]_{MeOH}+2^{o}$

The salt was cleaved as described in Example 2 to give D-3,4-dihydroxyphenylglycine.

Claims

1. A diastereoisomeric 3-bromocamphor-9-sulphonate salt of a dihydroxyphenyl glycine.

2. A salt as claimed in claim 1 having the formula I.

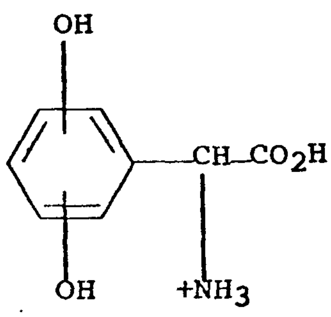

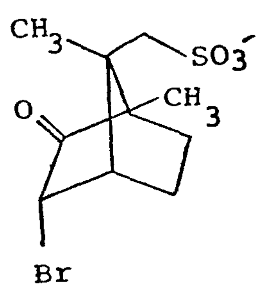

(I)

3. Diastereoisomeric 3-bromocamphor-9-sulphonate salts of 3,4 dihydroxyphenylglycine.

4. A process for the production of a 3-bromo camphor-9-sulphonate salt as claimed in claim 1 comprising treating a racemic dihydroxyphenyl-glycine of formula II.

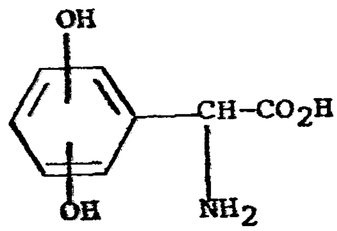

(II)

with an optically active 3-bromocamphor-9-sulphonic acid of formula III.

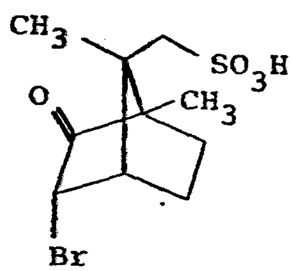

(III)

in a polar solvent.

5. A process as claimed in claim 4 in which the reactant is L(+)3 bromocamphor-9-sulphonic acid.

6. A process as claimed in claim 4 or claim 3 in which the solvent is water, a $C_{1-6}$ alkanoic acid or a dilute mineral acid.

7. A process as claimed in claim 6 in which the solvent is glacial acetic acid.

8. A process for the production of an optically active dihydroxyphenyl glycine comprising reacting a racemic dihydroxyphenylglycine with

an optically active 3-bromocamphor-9-sulphonic acid in a polar solvent to yield two diastereomeric salts, isolating one salt from the mother liquor by preferential crystallisation and liberating the optically active dihydroxyphenylglycine therefrom.

9. A process as claimed in claim 8 in which the polar solvent is water, a $C_{1-6}$ alkanoic acid or a dlute mineral acid.

10. A process as claimed in claim 9 in which the solvent is glacial acetic acid.

11. A process as claimed in any one of claims 8 to 10 in which the isolated salt is treated with a base to liberate the optically active dihydroxyphenylglycine.

12. A process as claimed in claim 11 in which the base is ammonia, sodium hydroxide ar potassium hydroxide.

13. A process as claimed in any one of claims 8 to 12 in which preferential crystallisation is induced by addition of a further solvent in which one diastereoisomer is less soluble.

14.. A process as claimed in any one of claims 8 to 12 in which preferential crystallisation is induced by the addition of a seed crystal of the desired diastereoisomer.

15. A process as claimed in any one of claims 8 to 14 in whch the second salt is subsequently recrystallised from the mother liquor by addition of a seed crystal of the diastereoisomeric salt remaining in solution.

16. A process as claimed in claim 15 in which the second salt is treated with a base to liberate an optically active dihydroxyphenylglycine and this is racemised to reform the DL-dihydroxyphenylglycine, whereafter a process as claimed in any one of claims 8 to 15 is repeated to give the desired diastereoisomer.

17. A process as claimed in claim 16 in which the optically active dihydroxyphenylglcyine is racemised by treatment with hydrochloric acid at 130 to 140°C.

18. A process as claimed in any one of claims 8 to 17 in which the reactant is L(+)3-bromocamphor-9-sulphonic acid whereby the salt with the D-enantiomer of the dihydroxyphenyl-glycine is crystallised.

19. A process as claimed in any one of claims 8 to 18 in which the dihydroxyphenylglycine is 3,4 dihydroxyphenylglycine.

20. D-3,4-dihydroxyphenylglycine when produced by a process as claimed in any one of claims 8 to 19.

European Patent Office

**EUROPEAN SEARCH REPORT**

**0 164 026**

EP 85106310.7

**DOCUMENTS CONSIDERED TO BE RELEVANT**

| Category | Citation of document with indication, where appropriate, of relevant passages | Relevant to claim | CLASSIFICATION OF THE APPLICATION (Int. Cl.4) |
|---|---|---|---|
| X | CHEMICAL ABSTRACTS, vol. 91, no. 3, July 16, 1979, Columbus, Ohio, USA<br>SH. YAMADA et al. "Preparation of D-p-hydroxyphenylglycine" page 708, column 2; page 709, column 1, abstract-no. 21 030d<br>& Agric. Biol. Chem. 1979, 43(2), pages 395-6.<br>-- | 1,4, 8,11, 12 | C 07 C 101/30<br>C 07 C 99/12<br>C 07 C 143/20<br>C 07 B 57/00 |
| X | PATENTS ABSTRACTS OF JAPAN, unexamined applications, section C, vol. 1, no. 111, September 26, 1977<br>THE PATENT OFFICE JAPANESE GOVERNMENT<br>page 2 525 C 77<br>* Kokai-no. 52-71 440 (TANABE SEIYAKU K.K.) *<br>---- | 1,4, 8,11, 12 | **TECHNICAL FIELDS SEARCHED (Int. Cl.4)**<br>C 07 C 99/00<br>C 07 C 101/00<br>C 07 C 143/00<br>C 07 B |

The present search report has been drawn up for all claims

| Place of search | Date of completion of the search | Examiner |
|---|---|---|
| VIENNA | 11-07-1985 | HOFBAUER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document